# FASCICULE DE BREVET EUROPEEN

(11) **EP 1 336 109 B1**
(45) Date de publication et mention de la délivrance du brevet: **04.07.2007**
(21) Numéro de dépôt: 01993830.7
(22) Date de dépôt: 08.11.2001
(51) Int. Cl.: G01N 33/74, G01N 33/68, C07K 16/22, C12N 5/12

(54) **TROUSSE ET PROCEDE DE DETECTION DE LA PROTEINE ESM-1**
KIT UND VERFAHREN ZUR BESTIMMUNG VON PROTEIN ESM-1
KIT AND METHOD FOR DETECTING THE ESM-1 PROTEIN

(30) Priorité: 09.11.2000 FR 0014421
(43) Date de publication de la demande: 20.08.2003
(73) Titulaire: INSTITUT PASTEUR DE LILLE, 59019 Lille cedex (FR); INSTITUT NATIONAL DE LA SANTE ET DE LA RECHERCHE MEDICALE (INSERM), 75654 Paris Cédex 13 (FR)
(72) Inventeur: LASSALLE, Philippe, F-59000 LILLE (FR); BECHARD, David, F-75010 PARIS (FR); TONNEL, André-Bernard, F-59840 PREMESQUES (FR)
(74) Mandataire: Michelet, Alain
(86) Numéro de dépôt international: PCT/FR2001/003477
(87) Numéro de publication internationale: WO 2002/039123

(56) Documents cités:
- FR-A- 2 775 691
- BECHARD DAVID ET AL: "Characterization of the secreted form of endothelial-cell-specific molecule 1 by specific monoclonal antibodies." JOURNAL OF VASCULAR RESEARCH, vol. 37, no. 5, septembre 2000 (2000-09), pages 417-425, XP001016197 ISSN: 1018-1172 cité dans la demande
- LASSALLE P ET AL: "ESM-1 IS A NOVEL HUMAN ENDOTHELIAL CELL-SPECIFIC MOLECULE EXPRESSED IN LUNG AND REGULATED BY CYTOKINES" JOURNAL OF BIOLOGICAL CHEMISTRY, AMERICAN SOCIETY OF BIOLOGICAL CHEMISTS, BALTIMORE, MD, US, vol. 271, no. 34, 23 août 1996 (1996-08-23), pages 20458-20464, XP002086198 ISSN: 0021-9258 cité dans la demande

## Description

### Domaine de l'invention

La présente invention concerne un nécessaire ou trousse de détection de la protéine ESM-1 dans un échantillon. Elle est également relative à un procédé de détection de la protéine ESM-1 dans un échantillon mettant en oeuvre un tel nécessaire ou une telle trousse.

La trousse et le procédé de détection de la protéine ESM-1 selon l'invention sont industriellement applicables notamment à la quantification de la protéine ESM-1 dans des échantillons biologiques, tout particulièrement d'échantillons biologiques provenant de patients dont on suspecte une altération des parois vasculaires endothéliales.

### Etat de la technique

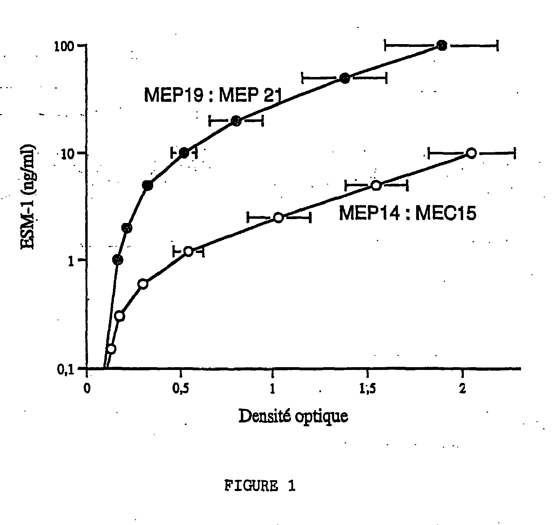

La protéine ESM-1 (pour « Endothelial-cell-Specific Molecule 1 ») est une protéine principalement exprimée par les cellules endothéliales et majoritairement par les cellules endothéliales vasculaires. La protéine ESM-1 a été décrite pour la première fois par LASSALLE et al. (1996). L'ARN messager de ESM-1 code pour un polypeptide de 184 acides aminés dont la séquence est décrite dans la figure 1 de l'article de LASSALLE et al. (1996).

La protéine ESM-1 retrouvée dans les lysats cellulaires des cellules endothéliales humaines possède un poids moléculaire apparent de 20 kDa. La forme sécrétée de la protéine ESM-1 possède un poids moléculaire apparent de 50 kDa, indiquant que la protéine ESM-1 sécrétée a subi des modifications post-traductionnelles.

L'utilisation d'anticorps monoclonaux pour détecter la protéine ESM-1 est décrite dans la demande de brevet français publiée sous le n°2.775.691 ainsi que dans l'article de BECHARD et. al. (2000). Ces anticorps monoclonaux ont été préparés par immunisation de souris avec un antigène constitué de la séquence allant de l'acide aminé en position 79 jusqu'à l'acide aminé en position 184 de la protéine ESM-1 qui a été fusionnée à la protéine GST (glutathione-S-transferase). Trois familles d'anticorps monoclonaux avaient été caractérisées, ces familles d'anticorps se fixant respectivement sur les déterminants antigéniques AgD1 79P-99C (anticorps MEP 01, MEP 06 et MEP 21), AgD2 119 S-139V (anticorps MEP 08 et MEP13) et AgD3 159G-184R(anticorps MEP 04, MEP 14 et MEP19).

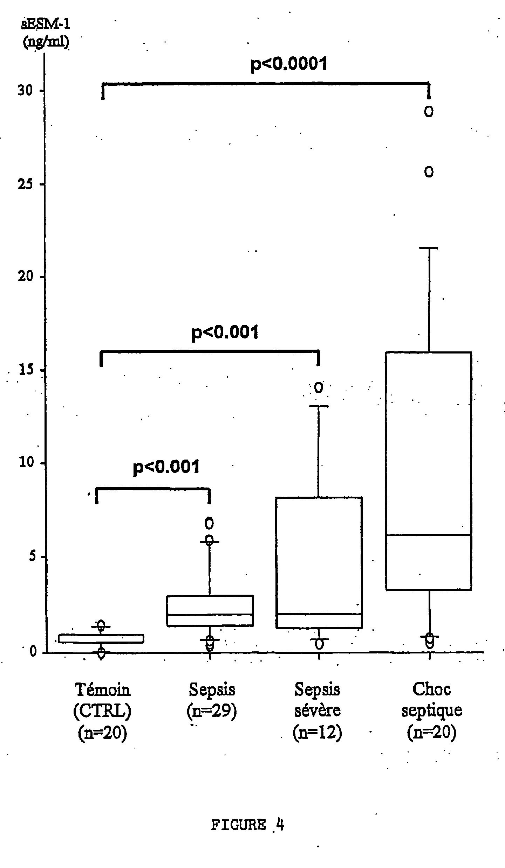

BECHARD et al.(2000) décrivent la mise au point d'un test immunoenzymatique du type « sandwich » mettant en oeuvre les anticorps monoclonaux MEP 19 et MEP 21 pour réaliser un test de détection de la protéine ESM-1 dans un échantillon. Ce test a permis aux auteurs de montrer une forte production de protéine ESM-1 dans le sérum de patients hospitalisés pour un choc septique. Les résultats présentés à la figure 4 de cet article montrent que le test de détection utilisant les anticorps MEP 19 et MEP 21 permet de détecter une concentration de protéine ESM-1 supérieure à 4 nanogrammes par millilitre.

Toutefois, le test immunoenzymatique de la protéine ESM-1 décrit par BECHARD et al. (2000), s'il est adapté à la détection de fortes concentrations de protéine ESM-1 dans un échantillon, ne permet pas de détecter de faibles quantités de cette protéine, qui sont pourtant significatives de troubles physiologiques, tels qu'une altération de la paroi vasculaire endothéliale chez des patients, comme cela est montré selon l'invention.

### SOMMAIRE DE L'INVENTION

Le demandeur s'est attaché à mettre au point un test immunologique de détection de la protéine ESM-1 dans un échantillon, beaucoup plus sensible que le test décrit dans l'état de la technique.

L'invention a pour objet un nécessaire ou trousse pour la détection de la protéine ESM-1 dans un échantillon, comprenant:
a) un premier anticorps se fixant spécifiquement sur la région N-terminale comprise entre l'acide aminé en position 1 et l'acide aminé en position 78 de la séquence en acides aminés de la protéine ESM-1; et
b) un second anticorps se fixant spécifiquement sur la région C-terminale comprise entre l'acide aminé en position 79 et l'acide aminé en position 184 de la séquence en acides aminés de la protéine ESM-1.

L'invention a également pour objet un procédé de détection de la protéine ESM-1 dans un échantillon, caractérisé en ce qu'il comprend les étapes suivantes:
a) mettre en contact l'échantillon à tester avec un support sur lequel est immobilisé un premier anticorps se fixant spécifiquement sur la région N-terminale ou la région C-terminale de la séquence en acides aminés de la protéine ESM-1;
b) mettre en contact le complexe éventuellement formé entre l'anticorps immobilisé et la protéine ESM-1 avec un second anticorps se fixant spécifiquement sur la région N-terminale ou C-terminale non reconnue par le premier anticorps; et
c) détecter le complexe éventuellement formé entre la protéine ESM-1 et le second anticorps.

L'invention est également relative à certains anticorps mis en oeuvre dans le procédé ci-dessus.

L'invention a encore pour objet l'utilisation de la trousse de détection selon l'invention pour la détection d'une altération de la paroi vasculaire endothéliale chez un patient, notamment chez un patient affecté d'un choc septique ou d'un cancer ou encore d'un patient subissant un traitement thérapeutique susceptible d'être cytotoxique vis-à-vis des cellules endothéliales vasculaires.

### DESCRIPTION DETAILLEE DE L'INVENTION

### TROUSSE DE DETECTION SELON L'INVENTION

II est fourni selon l'invention un nécessaire ou trousse permettant de détecter avec une grande sensibilité la présence de la protéine ESM-1 dans un échantillon.

L'échantillon dans lequel la présence de la protéine ESM-1 est recherchée peut être de toute nature. De préférence, il s'agit d'un fluide biologique tel qu'un sérum, un plasma, du sang total, de l'urine, un liquide cérébrospinal, ou des sumageants de culture ou encore des lysats cellulaires.

Le demandeur a montré que la protéine ESM-1 sécrétée par les cellules eucaryotes présentait des caractéristiques spécifiques qui n'étaient pas retrouvées dans la protéine ESM-1 produite par des cellules procaryotes, telles que les cellules de *Escherichia coli.*

La protéine ESM-1 est sécrétée par les cellules eucaryotes, et tout particulièrement par les cellules endothéliales, sous la forme d'une glycoprotéine, plus précisément un protéoglycane possédant une chaîne du type chondroïtine/sulfate de dermatane. De plus, le demandeur a montré que la protéine ESM-1 sécrétée par les cellules eucaryotes subissait d'autres modifications post-traductionnelles incluant des modifications conformationnelles provoquées par la formation de ponts disulfure entre les résidus cystéines localisés majoritairement dans la région N-terminale de cette protéine. Le polypeptide ESM-1 de 184 acides aminés est référencé comme la séquence SEQ ID N°1.

Pour la mise au point d'un test de détection immunologique de la protéine ESM-1 de grande sensibilité, le demandeur s'est attaché à préparer des anticorps monoclonaux dirigés contre la région N-terminale allant de l'acide aminé en position 20 à l'acide aminé en position 78 de la séquence SEQ ID N°1 décrite par LASSALLE et al. (1996), l'acide aminé en position 20 constituant l'acide aminé amino-terminal de la protéine ESM-1 sécrétée.

De préférence, les anticorps monoclonaux préparés par le demandeur sont dirigés contre la protéine ESM-1 sécrétée par des cellules eucaryotes et ayant subi les modifications post-traductionnelles précitées.

Plus spécifiquement, le demandeur a fabriqué des lignées cellulaires d'hybridomes produisant des anticorps monoclonaux se fixant spécifiquement sur la région de la protéine ESM-1 subissant des modifications conformationnelles du fait de la création de ponts disulfure, c'est-à-dire des anticorps dirigés contre la région N-terminale de la protéine ESM-1 allant de l'acide aminé en position 20 jusqu'à l'acide aminé en position 78 de la séquence en acides aminés de la protéine ESM-1 de séquence SEQ ID N°1.

Afin d'obtenir un outil de détection de la protéine ESM-1 dans un échantillon, le demandeur a mis au point un test immunologique du type « sandwich » mettant en oeuvre deux anticorps se liant spécifiquement à deux régions distinctes de la protéine ESM-1, respectivement un anticorps se liant spécifiquement à la région N-terminale allant de l'acide aminé en position 20 jusqu'à l'acide aminé en position 78 de ESM-1 et un anticorps se liant spécifiquement à la région C-terminale allant de la position 79 à la position 184 de la séquence de ESM-1.

Un premier objet de l'invention consiste donc en une trousse ou nécessaire de détection de la protéine ESM-1 dans un échantillon, ladite trousse comprenant:
a) un premier anticorps se liant spécifiquement à la région N-terminale de la protéine ESM-1 comprise entre l'acide aminé en position 20 et l'acide aminé en position 78 de la séquence en acides aminés de cette protéine; et
b) un second anticorps se liant spécifiquement à la région C-terminale comprise entre l'acide aminé en position 79 et l'acide aminé en position 184 de la séquence en acides aminés de la protéine ESM-1.

De manière tout à fait préférée, l'anticorps se liant spécifiquement à la région N-terminale de ESM-1 est dirigé spécifiquement contre la région N-terminale de la protéine ESM-1 sécrétée par les cellules eucaryotes et ayant subi les modifications post-traductionnelles détaillées ci-dessus. Cela signifie que l'anticorps se liant spécifiquement à la région N-terminale a été obtenu selon un procédé comportant une étape au cours de laquelle on injecte à un animal une préparation purifiée de la protéine ESM-1 produite par une cellule eucaryote.

Par « anticorps » au sens de l'invention, on entend toute molécule comprenant un « paratope » capable de se lier spécifiquement à la région N-terminale ou à la région C-terminale de la protéine ESM-1. Par « anticorps » selon l'invention, on entend aussi une population homogène de molécules comprenant toutes le même « paratope » capable de se lier spécifiquement à la région N-terminale ou C-terminale de la protéine ESM-1.

On entend par « paratope » le site de combinaison antigénique compris dans le fragment Fab d'un anticorps qui est localisé dans les régions hypervariables ou CDR des domaines variables V_{H} et V_{L} de la chaîne lourde et de la chaîne légère d'une immunoglobuline.

Un anticorps selon l'invention peut être préparé à partir d'hybridomes selon la technique décrite par KOHLER et MIELSTEIN (1975), ou encore par la technique de l'hybridome de cellules B humaines décrites par KOZBOR (1983). Un anticorps selon l'invention englobe également les fragments d'anticorps chimériques Fv simple chaîne (ScFv pour « Single Chain Fv ») tel que décrit dans le brevet US n°4.946.778 et par MARTINEAU et al.(1998). Un anticorps selon l'invention peut également être produit par des banques de phages (« Phage Display Libraries ») telles que décrites par RIDDER et al. (1995). Un anticorps selon l'invention peut être également un anticorps humanisé produit selon la technique décrite par REINMANN et al.(1997) ou encore par la technique décrite par LEGER et al.(1997).

De manière tout à fait préférée, la trousse de détection de la protéine ESM-1 selon l'invention comprend l'un des deux anticorps sous une forme immobilisée sur un support. Dans ce mode de réalisation préféré, la trousse de détection de l'invention se présente sous une forme dite « prête à l'emploi » pour réaliser un test d'immunodétection du type « sandwich » de la protéine ESM-1.

II a été montré selon l'invention que la mise en oeuvre d'une trousse telle que définie ci-dessus pour la détection de la présence de la protéine ESM-1 dans un échantillon permettait d'obtenir une sensibilité de détection 10 fois plus grande que celle observée avec le test de détection de ESM-1 décrit par BECHARD et al. (2000).

La haute sensibilité du test d'immunodétection selon l'invention a rendu possible la détection de concentrations très faibles de la protéine ESM-1 dans un échantillon biologique, par exemple dans un sérum humain, et a ainsi permis la détection précoce d'altération des parois vasculaires endothéliales chez un patient.

Notamment, la mise en oeuvre d'une trousse d'immunodétection selon l'invention a permis de détecter des concentrations sériques de ESM-1 allant de 1 à 3 nanogrammes par ml chez des individus dits « atopiques » atteints de sepsis .

Comme cela est montré dans les exemples, la mise en oeuvre d'une trousse d'immunodétection selon l'invention permet de détecter des concentrations de ESM-1 de l'ordre de 100 à 200 picogramme/ml, alors que le test décrit par BECHARD et al.(2000) ne permet pas la détection de concentrations de ESM-1 inférieures à 1 nanogramme/ml.

Avantageusement, l'anticorps dirigé contre la région N-terminale de ESM-1 est un anticorps monoclonal produit par une lignée d'hybridome obtenue après immunisation d'un mammifère, de préférence une souris, avec la protéine ESM-1 recombinante synthétisée par une cellule eucaryote, par exemple par une cellule de la lignée CHO transformée par un vecteur d'expression comprenant un insert d'ADN codant pour la protéine ESM-1 décrite par LASSALLE et al. , par exemple le vecteur pcDNA3.

De manière tout à fait préférée, il s'agit de l'anticorps monoclonal produit par la lignée d'hybridome désignée MEC 15 déposée à la Collection de Cultures de Micro-organismes (CNCM) de l'Institut Pasteur le 17 Octobre 2000 sous le N° d'accès I-2572.

L'anticorps MEC15 constitue un objet de l'invention.

L'anticorps se liant spécifiquement à la partie C-terminale de la protéine ESM-1 peut être indifféremment un anticorps dirigé contre la protéine ESM-1 produite par une cellule procaryote, telle que *Escherichia coli,* ou par une cellule eucaryote, telle que les cellules de la lignée CHO.

Préférentiellement, l'anticorps se liant spécifiquement à la région C-terminale de ESM-1 est choisi parmi les anticorps capables de reconnaître l'un des trois déterminants antigéniques suivants de la protéine ESM-1:
- le déterminant AgD1 allant du résidu proline en position 79 jusqu'au résidu cystéine en position 99 de ESM-1, tels que les anticorps produits par les lignées d'hybridome MEP01, MEP06 et MEP21 décrits par BECHARD et al. (2000) ;
- le déterminant antigénique AgD2 allant du résidu sérine en position 119 jusqu'au résidu valine en position 139 de ESM-1 reconnu par les anticorps produits par les hybridomes MEP08 et MEP13 décrits par BECHARD et al.(2000);
- le déterminant antigénique AgD3 allant du résidu glycine en position 159 jusqu'au résidu arginine en position 184 de ESM-1 est reconnu par les anticorps produits par les lignées d'hybridome MEP04, MEP14 ET MEP19 décrits par BECHARD et al. (2000).

De manière tout à fait préférée, l'homme du métier pourra avantageusement se référer, pour l'utilisation d'un anticorps se liant spécifiquement à la région C-terminale de la protéine ESM-1, aux anticorps suivants:
- les anticorps monoclonaux spécifiques du déterminant antigénique D1 produits par la lignée d'hybridome déposée auprès de la CNCM le 19 Novembre 1997 sous le N° d'accès I-1944 (anticorps MEP 21);
- les anticorps monoclonaux spécifiques du déterminant antigénique D2 produits par la lignée d'hybridome déposée le 19 Novembre 1997 auprès de la CNCM sous le N° d'accès I-1941 (anticorps MEP08);
- les anticorps monoclonaux spécifiques du déterminant antigénique D3 produits par la lignée d'hybridome déposée le 19 Novembre 1997 auprès de la CNCM sous le N° d'accès I-1942 (anticorps MEP14) et I-1943 (anticorps MEP19).

Dans le mode de réalisation préféré d'une trousse d'immunodétection selon l'invention, le premier et le second anticorps sont choisis de telle sorte que leurs sites respectifs de reconnaissance sur la protéine ESM-1 soient très distants l'un de l'autre afin d'éviter tout événement de fixation compétitive d'un anticorps vis-à-vis de l'autre sur la protéine, qui pourrait être provoqué par un phénomène d'encombrement stérique dans le cas où les sites de reconnaissance respectifs des deux anticorps sont trop voisins.

Ainsi, dans un mode de réalisation préféré d'une trousse de détection selon l'invention, le premier anticorps se liant spécifiquement sur la région N-terminale de la protéine ESM-1 est l'anticorps produit par la lignée d'hybridome MEC15. Selon ce mode de réalisation préféré, l'anticorps se liant spécifiquement à la région C-terminale de la protéine ESM-1 est l'anticorps monoclonal produit par la lignée d'hybridome MEP14.

De manière générale, un anticorps dirigé contre la région C-terminale de la protéine ESM-1 peut être sélectionné selon la technique décrite par BECHARD et al. (2000), qui consiste à préparer un vecteur d'expression comprenant un insert d'ADN codant pour la région C-terminale de la protéine ESM-1 fusionnée à la protéine GST, puis à immuniser des souris avec la protéine ESM-1 purifiée obtenue à partir de lysats cellulaires de cellules de *Escherichia coli* transformées avec le vecteur d'expression précité. Après fusion des cellules spléniques des souris ainsi immunisées avec des cellules de myélome afin d'obtenir une série d'hybridomes produisant des anticorps monoclonaux, les anticorps d'intérêt se liant spécifiquement à la région C-terminale de la protéine ESM-1 peuvent être sélectionnés par cartographie d'épitopes à l'aide de peptides dérivés de ESM-1 contenant des délétions progressives de l'extrémité N-terminale de la région C-terminale de cette protéine.

De même, l'homme du métier peut sélectionner des anticorps se liant spécifiquement à la région N-terminale de la protéine ESM-1 en immunisant des souris avec la protéine ESM-1 complète, de préférence la protéine ESM-1 complète produite par une cellule hôte eucaryote, puis en produisant une série de lignées d'hybridomes à partir des cellules spléniques des souris immunisées, puis à sélectionner les anticorps d'intérêt se liant spécifiquement sur la région N-terminale, par exemple par des tests d'immuno-détection compétitifs avec les anticorps se fixant spécifiquement sur la région C-terminale de ESM-1. Les anticorps d'intérêt sont ceux qui n'entrent pas en compétition avec les anticorps spécifiques de la région C-terminale de ESM-1, c'est-à-dire les anticorps qui n'entrent en compétition avec aucun des anticorps spécifiques des déterminants antigéniques D1, D2 et D3 de la protéine ESM-1.

De préférence, l'un des deux anticorps constitutifs de la trousse de détection selon l'invention est immobilisé sur un support. Le support sur lequel est immobilisé l'anticorps peut être de toute nature connue de l'homme du métier spécialiste des tests d'immuno-détection, en particulier des tests d'immuno-détection du type « sandwich ».

A titre illustratif, le support sur lequel est immobilisé l'anticorps peut être un matériau insoluble dans l'eau poreux ou non poreux. Le support peut être hydrophile ou susceptible d'être rendu hydrophile et comprend les poudres inorganiques telles que la silice, le sulfate de magnésium et l'aluminium; les matériaux polymères naturels, en particulier les matériaux cellulosiques et les matériaux dérivés de la cellulose; des polymères naturels ou synthétiques tels que la nitrocellulose, l'acétate de cellulose, le poly(chlorure de vinyle), le polyacrylamide, le dextran réticulé, l'agarose, le polyacrylate, le polyéthylène, le polypropylène, le poly(4-méthylbutène), le polystyrène, le polyméthacrylate, le poly(téréphtalate d'éthylène), le Nylon, le poly(butyrate de vinyle), certains types de.verre tels que le Bioglass, ou des céramiques.

La fixation d'un anticorps selon l'invention sur un support peut être réalisée par des techniques bien connues de l'homme du métier. Le support peut se présenter sous des formes diverses, y compris sous forme de bandes ou de particules telles que des bittes. La surface du support peut être polyfonctionnelle ou susceptible d'être polyfonctionnalisée de manière à fixer l'anticorps via des interactions covalentes ou non covalentes qui peuvent être spécifiques ou non spécifiques.

A titre illustratif, pour l'immobilisation d'anticorps sur un support, l'homme du métier peut avantageusement se référer au brevet US N°4,168,146 ou encore au brevet US N°4,347,311.

Avantageusement, l'un des anticorps constitutifs de la trousse de détection selon l'invention est lié de manière covalente à une molécule permettant sa détection directe, ou indirecte.

De préférence, dans le mode de réalisation dans lequel l'un des anticorps est immobilisé sur un support, l'autre anticorps est lié de manière covalente à une molécule permettant sa détection directe, ou indirecte.

La molécule détectable peut être isotopique ou non isotopique.

A titre illustratif, mais non limitatif, la molécule détectable peut intervenir dans une réaction catalytique, telle qu'une enzyme, un fragment d'enzyme, un substrat d'enzyme, un inhibiteur d'enzyme, un co-enzyme ou un catalyseur. La molécule détectable peut être également un chromogène, tel qu'un fluorophore, un colorant, une molécule chimiluminescente.

La molécule détectable peut ainsi être une molécule fluorescente telle que les molécules décrites par ICHINOSE et al. (1991) ou encore des dérivés d'isothiocyanate fluorescents, la phycoerithrine, l'isothiocyanate de rhodamine, le chlorure de dansyle ou encore le composé XRITC, la protéine GFP(« Green Fluorescent Protein ») du poisson Aequorea Victoria et ses nombreux dérivés, ou encore la protéine YFP (« Yellow Fluorescent Protein ») ainsi que la protéine luciférase.

Parmi les molécules détectables possédant une activité catalytique, les molécules préférées sont les enzymes suivantes, selon la Classification Internationale I.U.B.:(i) les oxydoréductases de classe 1 et (ii) les hydrolases de la classe 3. Les oxydoréductases préférées sont (i) les déhydrogénases de la classe 1.1, plus particulièrement 1.1.1-, 1.1.3 et 1.1.99 et (ii) les peroxydases de la classe 1.11 et (iii) des hydrolases de la classe 3.1, et plus particulièrement de la classe 3.1.3 et de la classe 3.2, plus particulièrement 3.2.1. Les déhydrogénases préférées sont la malate déshydrogénase; la glucose-6-phosphate déhydrogénase et la lactate déhydrogénase. L'oxydase préférée est la glucose oxydase. La peroxydase préférée est la peroxydase de raifort (« horse radish peroxydase »). Les hydrolases préférées sont les phosphatases alcalines, la β-gtucosidase et le lyzozyme.

La molécule détectable peut être également une molécule marquée radioactivement, par exemple par un isotope choisi parmi [³H], [³²P] et [¹²⁵I].

Dans le mode de réalisation dans lequel la molécule détectable constitue un marqueur indirect, l'un des anticorps constitutifs d'une trousse de détection selon l'invention peut être lié covalemment à un ligand telle que la biotine ou la streptavidine.

Dans ce mode de réalisation particulier, la molécule détectable est choisie de manière à ce qu'elle se fixe sur le ligand lié de manière covalente à l'anticorps. La molécule détectable peut par exemple être liée elle-même respectivement à la biotine ou à la streptavidine.

Selon encore un autre mode de réalisation d'une trousse de détection selon l'invention, le moyen de révélation de la formation d'un complexe entre la protéine ESM-1 présente dans l'échantillon testé et l'un des anticorps spécifiques de la protéine ESM-1, peut être un anticorps, par exemple un anticorps capable de se lier spécifiquement à la partie Fc de l'anticorps anti-ESM-1 ou encore un anticorps capable de se fixer spécifiquement à l'isotype auquel appartient l'anticorps anti-ESM-1 considéré, par exemple un anticorps reconnaissant spécifiquement les anticorps de souris de l'isotype IgG1.

Dans un mode de réalisation préféré d'un nécessaire de détection selon l'invention, c'est l'anticorps reconnaissant spécifiquement la région C-terminale de la protéine ESM-1 est immobilisé sur un support

Avantageusement, une telle trousse de détection est celle dans laquelle l'anticorps produit par l'hybridome MEP14 (CNCM N°I-1942) est immobilisé sur le support, est l'anticorps se liant spécifiquement sur la région N-terminale de la protéine ESM-1 et l'anticorps produit par l'hybridome MEC15 (CNCM N°I-2572).

L'outil de détection peut être alors un anticorps monoclonal biotinylé , par exemple de rat, reconnaissant spécifiquement les anticorps de souris de l'isotype IgG1, le système de détection pouvant être fourni par un conjugué streptavidine-peroxydase.

### PROCEDE D'IMMUNODETECTION SELON L'INVENTION

L'invention a encore pour objet un procédé de détection de la protéine ESM-1 dans un échantillon caractérisé en ce qu'il comprend les étapes suivantes:
a) mettre en contact l'échantillon à tester avec un premier anticorps se liant spécifiquement sur la région N-terminale de la protéine ESM-1 allant de l'acide aminé en position 20 à l'acide aminé en position 78 ou avec un premier anticorps se liant sur la région C-terminale de la protéine ESM-1.
b) mettre en contact le complexe éventuellement formé entre la protéine ESM-1 présente dans l'échantillon et le premier anticorps avec un second anticorps se liant spécifiquement à la région de la protéine ESM-1 choisie parmi la région N-terminale allant de l'acide aminé en position 20 à l'acide aminé en position 78 et la région C-terminale non reconnue par le premier anticorps; et
c) détecter le complexe formé entre la protéine ESM-1 et le second anticorps.

Par « complexe formé entre la protéine ESM-1 et le second anticorps », on entend le complexe formé entre le complexe formé entre :
- le complexe formé entre la protéine ESM-1 et le premier anticorps, et
- le second anticorps.

Selon un premier mode de réalisation du procédé d'immunodétection ci-dessus, le premier ou le seconde anticorps est immobilisé à la surface d'un support.

Selon un second mode de réalisation du procédé d'immuno-détection ci-dessus, l'anticorps qui est immobilisé sur un support est un anticorps se liant spécifiquement sur la région C-terminale de la protéine

ESM-1 et le second anticorps est un anticorps se liant spécifiquement sur la région N-terminale de cette protéine.

Selon un troisième mode de réalisation particulier du procédé d'immuno-détection, l'anticorps qui est immobilisé sur un support se lie spécifiquement sur la région N-terminale de la protéine ESM-1 allant de l'acide aminé en position 20 à l'acide aminé en position 78 et le second anticorps se lie spécifiquement sur la région C-terminale de cette protéine.

Les anticorps se liant spécifiquement sur les régions N-terminale ou C-terminale de ESM-1 sont tels que définis précédemment.

Dans un mode de réalisation préféré du procédé de détection selon l'invention, l'anticorps se liant spécifiquement sur la région N-términale de la protéine ESM-1 est l'anticorps MEC15 produit par l'hybridome déposé le 17 Octobre 2000 auprès de la CNCM sous le N° d'accès I-2572.

Le second anticorps peut être l'anticorps MEP14 produit par l'hybridome déposé à la CNCM le 19 Novembre 1997 sous le N°I-1942.

L'étape c) du procédé ci-dessus peut être réalisée à l'aide d'un anticorps biotinylé capable de se fixer au second anticorps, la molécule détectable pouvant être constituée d'un conjugué du type streptavidine-peroxydase.

### APPLICATIONS DU PROCEDE DE DETECTION SELON L'INVENTION.

Le demandeur a montré que le procédé d'immunodétection de la protéine ESM-1 selon l'invention permettait de quantifier avec une grande sensibilité la concentration sérique de protéine ESM-1 chez l'homme, en particulier chez des patients atteints de sepsis, et a permis d'établir une corrélation entre la quantité de protéine ESM-1 circulante et la gravité du sepsis chez les patients.

Selon la norme définie par l'American College of Chest Physicians/Society of Critical Care Medicine Consensus Conférence (1992, definitions for sepsis and multiple organ failure, and guide lines for the use of innovative therapies in sepsis; Crit Care Med., vo1.20: 864-874), le sepsis peut être divisé en trois catégories de gravité croissante: le sepsis, le sepsis sévère et le choc septique.

Grâce au procédé d'immunodétection selon l'invention, il a été établi une corrélation entre la gravité du sepsis et la concentration de protéine ESM-1 circulante chez les patients.

De plus, la grande sensibilité du test d'immunodétection selon l'invention a permis de montrer que les patients atteints d'un sepsis simple possédaient une concentration de protéine ESM-1 circulante laquelle, bien que faible, était significativement détectable et significativement supérieure (p < à 0.001) à la concentration de protéine ESM-1 retrouvée dans le sérum de volontaires sains.

Jusqu'à présent, l'évolution du sepsis chez un patient était suivie par la quantification de trois marqueurs, respectivement la protéine C-réactive, la protéine ICAM-1 soluble et la pro-calcitonine.

Il a été montré selon l'invention que l'évolution des taux de protéine C-réactive et de protéine ICAM-1 soluble ne corrélaient pas avec l'évolution de la concentration de ESM-1.

En revanche, il existe une bonne corrélation entre l'évolution des taux de pro-calcitonine et de la protéine ESM-1. Toutefois, la signification biologique de la pro-calcitonine dans le cas du sepsis n'est pas connue et cette protéine ne constitue donc pas un bon marqueur biologique de l'évolution du sepsis.

Au contraire, la protéine ESM-1, du fait de son rôle dans la régulation des réactions inflammatoires, constitue un nouveau marqueur de l'évolution du sepsis dont la signification physiologique est liée directement au développement non contrôlé de la réaction inflammatoire, particulièrement du recrutement des leucocytes pendant les phénomènes d'extravasation et d'infiltration massive de ces cellules dans les différents tissus, notamment les tissus pulmonaires, phénomènes causals, ou à tout le moins concomittants, avec une altération de la paroi vasculaire endothéliale.

Ainsi, le demandeur a montré que le test d'immunodétection de l'invention permettait la mise en évidence de faibles concentrations de protéine ESM-1 circulante, de l'ordre de 1 à 3 nanogrammes par millilitre, ces faibles concentrations retrouvées chez les patients atopiques pouvant être clairement distinguées des concentrations basales de l'ordre du nanogramme/ml ou même inférieure mesurée chez les volontaires sains.

De plus, il a été montré que la quantité de ESM-1 sérique retrouvée chez les patients atteints de sepsis constituait un pronostic fiable de mortalité chez ces patients. Ainsi, sur 68 patients testés, les patients ayant décédé 10 jours après leur hospitalisation présentaient des concentrations de ESM-1 circulantes au jour 0 et au jour 1 significativement supérieures aux concentrations de ESM-1 retrouvées dans le sérum des patients ayant survécu (10,6 ± 0,8 nanogrammes par ml chez les patients décédés contre 4,0 ± 0,6 nanogrammes par ml chez des patients ayant survécu [p > 0,01]).

Cette corrélation étroite entre le niveau circulant de protéine ESM-1 et le pronostic de mortalité chez les patients n'est pas retrouvée avec des marqueurs classiques du sepsis, c'est-à-dire la protéine C-réactive, la procalcitonine et la protéine ICAM-1 soluble.

Toutefois, la mesure de la concentration de ESM-1 circulante chez un patient ne suffit pas elle seule à réaliser un diagnostic médical permettant au médecin de prendre les mesures thérapeutiques adaptées à l'état global du patient

Un autre objet de l'invention consiste en l'utilisation d'une trousse de détection de la protéine ESM-1 selon l'invention pour détecter *in vitro* des altérations de la paroi vasculaire endothéliale chez l'homme, en particulier chez des patients.

L'invention est également relative à l'utilisation d'une trousse de détection de la protéine ESM-1 telle que définie ci-dessus pour le suivi *in vitro* d'un marqueur de la gravité d'un sepsis chez un patient.

II a aussi été montré selon l'invention qu'une concentration de protéine ESM-1 circulante supérieure à la normale était retrouvée chez des patients ayant subi une greffe d'organes et traités par un composé immunosuppresseur. Chez ces patients greffés et traités par un composé immunosuppresseur. L'augmentation de la concentration de la protéine ESM-1 circulante est indicative d'une activité cytotoxique du composé immunosuppresseur vis-à-vis des cellules endothéliales, particulièrement des cellules endothéliales de la paroi vasculaire.

Ainsi, la mise en oeuvre d'une trousse d'immunodétection de la protéine ESM-1 selon l'invention rend possible le suivi de patients traités par des composés immunosuppresseurs et la détermination du moment où ces immunosuppresseurs deviennent cytotoxiques.

La détection de taux de ESM-1 circulante supérieure à la normale chez ces patients peut constituer une indication pour le médecin de nature à lui permettre de modérer les doses de composés immunosuppresseurs administrés, lorsque cette mesure est associée à d'autres paramètres physiologiques du patient.

Selon un autre aspect, l'invention concerne l'utilisation d'une trousse d'immunodétection selon l'invention pour la quantification de la protéine ESM-1 *in vitro* chez un patient traité par un composé immunosuppresseur, en particulier chez un patient ayant subi une greffe d'organe.

Le demandeur a également montré une augmentation significative du taux de protéine ESM-1 circulante chez des patients atteints de cancer, en particulier de cancer broncho-pulmonaire.

Selon encore un autre aspect, l'invention concerne l'utilisation d'une trousse d'immunodétection telle que définie ci-dessus pour la quantification *in vitro* de la protéine ESM-1 chez un patient affecté d'un cancer.

De manière générale, un taux de protéine ESM-1 circulante supérieure à 1 nanogramme par ml est significatif d'une altération des cellules endothéliales de la paroi vasculaire et peut ainsi constituer un paramètre à prendre en compte lors de l'établissement d'un diagnostic clinique d'un patient donné par un médecin, étant entendu que ce seul paramètre, pris isolément, ne permet en lui-même d'établir aucun diagnostic thérapeutique ou clinique d'un patient.

La présente invention est en outre illustrée, sans être pour autant limitée, aux figures et aux exemples suivants:

### FIGURES

La figure **1** illustre une comparaison de la sensibilité de détection du test d'immuno-détection de la protéine ESM-1 selon l'invention (cercles vides) avec le test de détection décrit par BECHARD et al. (2000) représenté par les cercles pleins. L'échelle des ordonnées représente la concentration de la protéine ESM-1 dans l'échantillon à tester, exprimée en nanogrammes par ml. Sur l'échelle des abscisses, est représentée la densité optique.

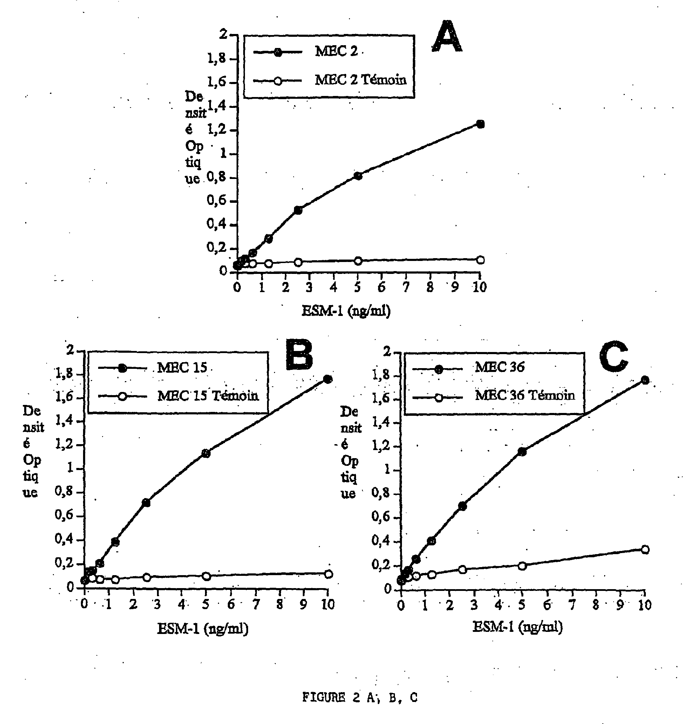

La figure **2** illustre les profils d'immunodétection réalisés selon la technique immunoenzymatique de type « sandwich ». Dans tous les cas, l'anticorps monoclonal produit par l'hybridome MEP14 est adsorbé sur une plaque de micro-titration. Sur l'échelle des abscisses est figurée la valeur de densité optique. Sur l'échelle des ordonnées , est représentée la concentration en protéine ESM-1 produite par les cellules de la lignée HEK293, exprimée en nanogrammes par ml. Le second anticorps utilisé dans le test d'immunodétection est un anticorps dirigé spécifiquement contre la région N-terminale de la protéine ESM-1. Il s'agit de l'anticorps MEC2 (figure 2A), MEC15 (figure 2B) et MEC36 (figure 2C).
La figure **3** illustre une comparaison de la concentration de protéine ESM-1 retrouvée dans le plasma ou le sérum de volontaires sains ou de patients. L'échelle des ordonnées représente la concentration sérique de la protéine ESM-1, exprimée en nanogramme par ml. L'échelle des abscisses représente la concentration plasmatique de la protéine ESM-1, exprimée en nanogramme par ml.
La figure **4** illustre la quantité de protéine ESM-1 circulante retrouvée dans le sérum de volontaires sains ou de patients atteints de sepsis de gravité clinique croissante. La concentration de protéine ESM-1 sérique, exprimée en nanogramme par ml, est représentée en ordonnée. En abscisse sont représentées les différentes populations d'individus testés, respectivement les sujets sains (CTRL), les patients atteints de sepsis, de sepsis sévère ou de choc sceptique, selon la définition clinique publiée dans Crit. Care Med., 1992, vol.20:864-874.
La figure **5** illustre la quantification de trois marqueurs du sepsis chez des populations de patients sains, ou de patients atteints de sepsis, de sepsis sévère ou de choc sceptique.
La figure **5A** illustre la quantification de protéine ICAM-1 soluble dans le sérum, exprimée en nanogramme par ml.
La figure **5B** illustre la quantification de protéine C-réactive, exprimée en milligramme par ml.
La figure **5C** illustre la quantification de procalcitonine sérique, exprimée en nanogramme par ml.
La figure **6** illustre une comparaison entre les niveaux de protéine ESM-1 circulante, et de chacun des trois marqueurs classiques du sepsis, respectivement la protéine ICAM-1 soluble, la protéine C-réactive et la procalcitonine au jour 0 d'hospitalisation des patients, respectivement chez les patients ayant survécu et chez les patients ayant décédé.
La figure **6A** illustre le niveau sérique de protéine ESM-1, exprimé en nanogramme par ml.
La figure **6B** illustre le niveau sérique de protéine ICAM-1 soluble, exprimé en nanogramme par ml.
La figure **6C** illustre le niveau de protéine C-réactive, exprimé en nanogramme par ml.
La figure **6D** illustre le niveau de procalcitonine, exprimé en nanogramme par ml.
La figure **7** illustre la valeur pronostic de la quantité de protéine ESM-1 ou de la protéine soluble ICAM-1 chez des patients au jour 0 et au jour 1 après l'hospitalisation, vis-à-vis de leur mortalité.
La figure **7A** illustre le niveau de protéine ESM-1, exprimé en nanogramme par ml retrouvé respectivement chez les patients ayant survécu (cercle plein) et les patients ayant décédé (cercle vide), respectivement au jour 0 et au jour 1 après l'hospitalisation.
La figure **7B** illustre le niveau de protéine soluble ICAM-1 sérique chez les mêmes patients.
La figure 8 illustre la valeur pronostic de mortalité du niveau de protéine ESM-1 et ICAM-1 soluble mesurée au jour 0 et au jour 1 après l'hospitalisation.
La figure **8A** illustre le niveau de protéine sérique, exprimé en nanogramme par ml, chez les patients ayant survécu (cercle plein) et chez les patients ayant décédé (cercle vide).
La figure 8B illustre le niveau de protéine soluble ICAM-1 sérique, exprimé en nanogramme par ml, chez les patients ayant survécu (cercle plein) et chez les patients ayant décédé (cercle vide).

### EXEMPLES

### EXEMPLE 1: Préparation des anticorps monoclonaux se liant spécifiquement sur la région N-terminale de la protéine ESM-1 produite par les cellules eucaryotes.

Afin d'obtenir des anticorps monoclonaux anti-ESM-1 dirigés contre la région N-terminale de la protéine ESM-1 riche en résidus cystéine, la forme native de la protéine ESM-1 produite par la lignée cellulaire CHO transfectée par un vecteur d'expression contenant un insert d'ADN codant pour la protéine ESM-1 a été purifiée.

La séquence d'ADNc codant pour la protéine ESM-1 est référencée comme la séquence SEQ ID N°2 du listage de séquences.

l'ADNc de ESM-1 est inséré dans le vecteur d'expression eucaryote pcDNA3 (In vitrogen) puis transfecté dans les cellules CHO avec la lipofectamine (Gibco) selon les recommendantuions du fabricant. 48 h après la transfection les cellules sont repiquées en présence d'un agent de selection (G418, Gibco) à la dose de 1000 microgramme / ml). Après deux semaines de sélection les cellules CHO résistantes au G418 sont clonées par dilution limite. Des clones exprimant ESM-1 sont ensuite sélectionnés et appelés CHO-ESM (déposé à la CNCM).

Pour la production, les cellules CHO-ESM sont cultivées en suspension dans un milieu conditionné sans serum de veau foetal (milieu CHO SFM II, Gibco). Le surnageant est ajusté à pH 8 et passé sur une colonne de DEAE-sépharose (Pharmacia). La colonne est lavée avec un tampon 50 mM Tris, pH 8, 0.2 M NaCl. La pmolécule ESM-1 est éluée dans un tampon 50 mM Tris, pH 8, 1 M NaCl. L'éluat est ensuite dilué au 1 :4 dans un tampon 50 mM Tris, pH 8 et incubé en présence d'anticorps monoclonal anti-ESM-1 (MEC4) immobilisé sur agarose (Biorad). Après une nuit d'icubation à 4°C sous agitation, les billes d'agarose sont lavées avec le tampon 50 mM Tris, pH 8, 0.2 M NaCl. ESM-1 est élué avec 3 M MgCl2. L'éluat est concentré et dialysé dans le tampon 50 mM Tris, pH 8, 0.5 M NaCl et stocké à -70°C.

Des souris Balb/C ont été immunisées par injection de 10 µg de protéine ESM-1 recombinante purifiée par souris, selon un protocole d'immunisation standard en présence d'adjuvant de Freund.

Les cellules d'hybridomes sécrétant les anticorps monoclonaux anti-ESM-1 ont été obtenues par fusion, criblage et sous-clonage selon la technique décrite par BECHARD et al. (2000).

Cinq clones cellulaires d'hybridome ont été obtenus et ont été désignés génériquement MEC (« Mouse Monoclonal Antibody to ESM-1 produced by CHO Cells »).

Quatre des hybridomes sélectionnés sont d'isotype IgG1,k respectivement les hybridomes désignés MEC4, MEC5, MEC15 et MEC36.

L'un des hybridomes est l'isotype IgM,k, l'hybridome MEC11.

Les clones cellulaires d'hybridomes ont été cultivés dans du milieu de culture en l'absence de sérum et les anticorps anti-ESM-1 ont été purifiés par chromatographie sur colonne de protéine G-Sépharose commercialisée par la Société Pharmacia (UPSALA, Suède).

### EXEMPLE 2: Sélection des anticorps monoclonaux dirigés spécifiquement contre la partie N-terminale de la protéine ESM-1 produite par les cellules de la lignée CHO.

Les anticorps MEC, de classe IgG1,K sont sélectionnés par la persistance d'une liaison forte des anticorps de la série MEC à ESM/Fc en présence d'anticorps MEP d'isotype différent et à la concentration finale de 1 µg/ml. Les tests sont réalisés par ELISA selon les exemples précédemments décrits pour la sélection des anticorps MEP et MEC, (notamment par les expériences d'immuno-détection par compétition décrites par LASSALLE et al. (1996).

### EXEMPLE 3: Test d'immuno-détection de la protéine ESM-1 selon l'invention.

Le test d'immuno-détection consiste en un test immunoenzymatique du type « sandwich » dont les caractéristiques générales sont identiques à celui décrit par BECHARD et al. (2000).

L'anticorps monoclonal anti-ESM-1 produit par la lignée d'hybridomes MEP14 (CNCM N°I-1942) a été dilué à la concentration de 5µg/ml dans un tampon carbonate 0,1 M, pH 9,5, et adsorbé pendant une nuit à +4°C sur une plaque 96 puits (plaque E.I.A./R.I.A., Costar, Cambridge, MA, USA).

La plaque a été saturée pendant une heure à la température du laboratoire avec un volume de 200 µl/puits de tampon PBS contenant 0,1% de sérum albumine bovine et 5mM de EDTA, puis lavée deux fois avec un tampon ELISA (le tampon PBS ci-dessus additionné de 0,1% de Tween 20).

Un calibrage a été réalisé avec la protéine ESM-1 purifiée selon la technique décrite par BECHARD et al. (2000).

Les échantillons de sang ont été dilués en série (1:2 à 1:128), dans un tampon ELISA et incubés sur une plaque ELISA pendant une heure à la température du laboratoire.

Les puits ont été lavés trois fois avec un tampon ELISA puis incubés pendant 1 heure à la température du laboratoire avec un second anticorps monoclonal dirigé contre ESM-1, l'anticorps MEC15 (CNCM N°I-2572) à la concentration de 0,1µg/ml dans 100 µl de tampon par puits.

Après trois lavages, on a ajouté un anticorps monoclonal de rat biotinylé dirigé contre les IgG1 de souris (commercialisé par PHARMINGEN) dilué dans un tampon ELISA et laissé incuber ce second anticorps pendant une heure.

Après trois lavages dans le tampon ELISA, les puits ont été incubés avec un conjugué de streptavidine-peroxydase à la dilution 1:10.000 v/v (commercialisé par la Société ZYMED).

Lèpres 30 minutes d'incubation avec le conjugué streptavidine-peroxydase, on réalise trois lavages de chaque puits dans un tampon ELISA puis deux lavages dans un tampon. PBS.

Le conjugué streptavidine-peroxydase est révélé avec le substrat TMB commercialisé par la Société SIGMA (Saint-Louis, MO, USA) en présence de 5 µl de H₂O₂ pendant 30'.

La réaction de révélation est stoppée par l'addition d'un volume de 100µl de H₂SO₄ 2N.

La plaque est lue à l'aide d'un spectrophotomètre (anthos labtec LP40, France) à la longueur d'onde de 405 nanomètres.

La concentration de la protéine ESM-1 plasmatique ou sérique est calculée à partir des mesures de densité optique et exprimée en nanogramme par ml.

### EXEMPLE 4:

### Résultats comparatifs de dosage de ESM-1 avec le test d'immuno-détection selon l'invention et un test d'immuno-détection de l'état de la technique.

Les dosages comparatifs de concentration connue de la protéine ESM-1 recombinante produite dans la lignée cellulaire CHO transfectée, puis purifiée comme décrit à l'exemple 1 , ont été réalisés.

Le premier dosage a été effectué conformément à l'enseignement de l'exemple 3. Brièvement, l'anticorps MEP14 a été immobilisé sur une plaque 96 puits du type ELISA, puis mis en contact avec une série de solutions tampons PBS contenant une concentration connue de protéine de ESM-1. Après lavage, le complexe formé entre l'anticorps MEP14 immobilisé et la protéine ESM-1 a été incubé avec une solution tampon contenant l'anticorps MEP 15 de l'invention.

Après une nouvelle série de lavages, le complexe antigène/anticorps formé a été incubé successivement avec un anticorps de rat anti-IgG1 de souris biotinylé, puis avec un conjugué streptavidine-peroxydase avant révélation par le peroxyde d'hydrogène.

Le second test a été réalisé dans les mêmes conditions, mais en utilisant l'anticorps MEP19 immobilisé sur la plaque 96 puits de type ELISA et l'anticorps MEP21 en tant que second anticorps dans cette technique immunoenzymatique du type «sandwich».

Les résultats sont présentés sur la figure 1.

Avec la combinaison d'anticorps selon l'invention, une différence significative dont la valeur de densité optique est obtenue avec une concentration de la protéine ESM-1 de l'ordre de 0,15 à 0,2 nanogrammes par millilitre.

En revanche, avec la combinaison des anticorps MEP19 et MEP21, une augmentation significative de la valeur de densité optique n'est obtenue que pour une concentration de ESM-1 égale à 1 ou 2 nanogrammes par ml.

Les résultats présentés à la figure 1 montrent clairement la grande sensibilité du test d'immunodétection de la protéine ESM-1 selon l'invention, par rapport à un test d'immunodétection de l'état de la technique, puisqu'on observe une différence de sensibilité comprise entre 5 et 10 fois entre ces deux tests, en faveur du test d'immunodétection de l'invention.

### EXEMPLE 5:

### Résultats comparatifs de tests d'immunodétection mettant en oeuvre différents anticorps de l'invention dirigés contre la région N-terminale de la protéine ESM-1 produite par les cellules de la lignée HEK293 transfectées.

Des tests d'immunodétection de type « sandwich » ont été réalisés conformément au protocole décrit à l'exemple 3.

Dans tous les cas, l'anticorps MEP14 a été immobilisé sur les puits d'une plaque 96 puits de type ELISA. Après incubation de la plaque ainsi préparée avec des solutions de concentrations connues de ESM-1 recombinantes produites par des cellules de la lignée HEK293 transfectées et lavage, le complexe antigène/anticorps formé a été incubé en présence d'un second anticorps, respectivement l'anticorps MEC2, l'anticorps MEC15 et l'anticorps MEC36.

Les résultats sont représentés à la figure 2.

Les résultats montrent que l'utilisation d'anticorps monoclonaux dirigée contre la région N-terminale de la protéine ESM-1 recombinante produite par des cellules eucaryotes dans un test immunoenzymatique de type « sandwich » permet un dosage de la protéine ESM-1 de très grande sensibilité, et dont la sensibilité est reproductible d'un anticorps à l'autre.

Pour l'ensemble des anticorps MEC15, MEC2 ET MEC36 testés, des concentrations de l'ordre de 0,15 à 0,2 nanogrammes par ml de ESM-1 ont pu être détectées.

### EXEMPLE 6: Application du test d'immuno-détection selon l'invention pour quantifier la protéine ESM-1 circulante chez des patients atteints de formes plus ou moins graves de sepsis.

### A. Matériels et méthodes.

### A.1 Test d'immuno-détection

Le test d'immunodétection a été réalisé conformément au protocole décrit à l'exemple 3 ci-dessus.

### A.2. Test des autres marqueurs sanguins

Le test quantitatif de la protéine soluble ICAM-1 sérique a été réalisé avec un test ELISA commercial (Diaclone Research, Besançon, France) La plaque est lue à l'aide d'un spectrophotomètre (Anthos Labtec LP40, France) à la longueur d'onde de 450 nanomètres. La concentration de ICAM-1 soluble sérique a été calculée à partir des mesures de la densité optique et exprimée en nanogramme par ml. La valeur normale de concentration de protéine ICAM-1 soluble a été de 571 ± 168 nanogrammes par ml (219-1042; n = 77).

Le test de quantification de la protéine procalcitonine a été réalisé avec un test immunologique du type ILMA (Lumitest, B.R.A.M.S.-Diagnostica GmbH, Allemagne).

La valeur normale de procalcitonine est inférieure à 0,5 nanogramme par ml. Les valeurs mesurées pour les patients atteints de syndrome de réponse inflammatoire systémique (SIRS pour « Systémic Inflammatory Response Syndrom ») sont généralement comprises entre 0,5 et 2 nanogrammes par ml.

La quantité de protéine C réactive a été mesurée par immuno-néphélométrie. La valeur normale est inférieure à 10 mg/l (Morley et al., 1982, Ann. N.Y. Acad. Sci., vol.389:406-418).

### A.3 Sujets

L'étude a été réalisée avec quatre groupes de sujets, un groupe de sujets sains non atopiques (rapport de sexe égal à 1) et trois groupes de patients atteints de troubles inflammatoires septiques systémiques et/ou pulmonaires (rapport de sexe égal à 1,6, âge : 56 ± 2 ans).

Tous les volontaires sains non atopiques présentaient une réponse négative au test cutané de réaction d'hypersensibilité immédiate, appelé aussi « prick test », et ne présentaient pas non plus d'historique clinique d'allergies.

Aucun des 68 patients n'était traité par des composés corticostéroïdes ou ne subissaient d'hémodialyse au moment du recueil d'échantillons de sang.

Le protocole d'expérimentation a été approuvé par le Comité Locale d'Hétique des Hôpitaux d'universités de Suisse.

### A.4 Définitions.

Les termes suivants ont été utilisés pour l'étude.
***Infection*** est un phénomène microbien caractérisé par une réponse inflammatoire à la présence de micro-organismes ou à l'invasion de tissu de l'hôte normalement stérile par ces micro-organismes.
***Syndrome de réponse inflammatoire systémique (SIRS pour « Systemic Inflammatory Response Syndrome »)*** avec aucun signe d'infection, est caractérisé par la présence d'au moins deux des quatre critères cliniques suivants:
a) fièvre ou hypothermie (température supérieure à 100,4°F [< à 38°C] ou > à 96,8°F[> à 36°C]);
b) tachycardie (> à 90 battements par minute);
c) tachypnée (> à 20 respirations par minute ou PaCO₂ < à 4,3 kPa[32 mm Hg]); et
d) un compte anormal de globules blancs > à 12.000 cellules/mm³; < à 4000 cellules / mm³, où la présence de plus de 10% de formes immatures respectivement.
***Sepsis*** est défini comme le SIRS accompagné d'une infection.
***Sepsis sévère*** est défini comme un sepsis associé avec un dysfonctionnement, une hypoperfusion ou une hypotension d'un organe. Des anormalités dans l'hypoperfusion et la perfusion peuvent inclure, mais ne sont pas limitées à l'oligurie (volume excrété < à 30 ml/hr), acidose lactique (niveau de lactate sérique supérieur à 2 mmol/L) où une altération aiguë du statut mental sans sédation (réduction d'au moins 3 points par rapport à la valeur de base selon le score de coma du type Glasgow).
***Choc septique*** est défini comme la présence d'un sepsis accompagné par une réduction durable de la pression sanguine cystolique (< à 90mmHg, ou une réduction de 40 mm Hg par rapport à la valeur de base de la pression sanguine cystolique), associé à la présence d'anormalités de perfusions (voir ci-dessus), en dépit d'une réanimation adéquate et le besoin d'amines vasoactives pour maintenir une pression sanguine adéquate (Crit. Care Med. 1992, vol.20:864-874).

### A.5 Description de l'étude

Pour chaque patient, les paramètres biologiques et cliniques suivants ont été obtenus: âge, sexe, diagnostic principal et secondaire tel que défini dans Crit. Care ed., 1992, vol.20: 864-874, antécédent, traitement, état vital 10 jours avant le recueil d'échantillons de sang, concentrations sérique et plasmatique de la protéine ESM-1.

Pour la plupart des patients, on a mesuré également d'autres paramètres biologiques sanguins tels que la ICAM-1 soluble circulante (sICAM-1.), la procalcitonine (proCT), et la protéine C-réactive (CRP).

Les échantillons de sang ont été recueillis de chacun des patients (ou de chacun des volontaires sains) dans des tubes secs et dans des tubes traités à l'EDTA, puis centrifugés pendant 30 mn à la vitesse de 3500 tr/m dans une centrifugeuse du type jouan C3i(France).

Les échantillons de sérum et de plasma ont été conservés pendant une courte durée à -20°C avant la réalisation des tests.

La période de survie à dix jours de l'hospitalisation a été choisie en postulant que l'infection avait contribué directement à la mort chez ces patients ou que la contribution du trouble inflammatoire initial par rapport à d'autres causes de mortalité ne pouvait pas être exclue.

### A.7 Analyse statistique

Les données sont exprimées comme la moyenne plus ou moins l'erreur standard (SEM).

Les comparaisons des niveaux moyens de ESM-1, sICAM-1, procalcitonine et CRP entre les différents groupes ont été réalisées en utilisant le test « One way Analysis » Anova (Bonferroni-Dunn), le test de Kruskal-Wallis et le test de Mann-Whitney.

La corrélation entre les valeurs des marqueurs a été réalisée grâce au test Spearman Rank.

La corrélation entre le niveau de marqueur circulant et la gravité de l'issue pour le patient a été réalisée en utilisant le test « One Way » ANOVA (Bonferroni-Dunn), le test de Mann-Whitney et le test de Wilcoxon.

En général, une valeur de p < à 0,05 est considérée comme statistiquement significative.

Les graphes ont été réalisés sous la forme de graphe en boîtes verticales représentés par leur médiane et les premier et troisième espaces interquartiles.

### B. RESULTATS

### B.1 Corrélation entre les niveaux plasmatiques et sériques de ESM-1.

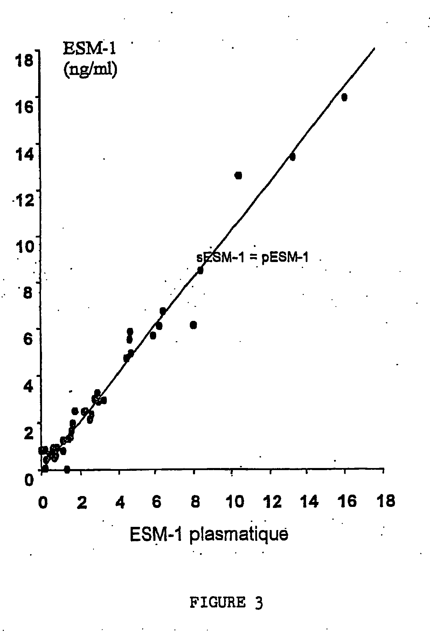

Dans un premier essai, on a déterminé si les conditions de recueil d'échantillons de sang pouvaient affecter la valeur du niveau de ESM-1. La comparaison des valeurs de niveaux de ESM-1 plasmatiques et sériques chez 41 sujets sains ont montré une forte corrélation (r = 0,85; p < à 0,0001). Dans la figure 3, on peut observer que, comme pour le groupe témoin, aucune différence significative n'est observée pour un patient donné entre les niveaux plasmatiques et sériques de ESM-1, sur les premiers trente six patients de l'étude.

Ces résultats suggèrent tout d'abord que la coagulation du sang ne modifie pas le niveau de ESM-1. Ces résultats suggèrent aussi que la protéine ESM-1 peut être quantifiée indifféremment à partir d'échantillons de sang ou de plasma.

En conséquence, seul le niveau sérique de ESM-1 a été utilisé pour le reste de l'étude.

### B.2 Groupe contrôle

Une valeur moyenne du niveau sérique de ESM-1 était de 0,7 ± 0,4 nanogrammes par ml chez les sujets sains, comme illustré dans le tableau 1, alors que le niveau sérique de ESM-1 était de 1,0 +/- 0,1 nanogramme par ml dans le groupe de sujets atopiques.

Bien que la différence soit faible, elle est statistiquement significative (p < à 0,05).

On conséquence, le groupe contrôle auquel il est fait référence dans l'étude est le groupe des sujets sains non atopiques représentatifs de la population générale, et la valeur moyenne du niveau de ESM-1 dans ce groupe de sujets sains non atopiques a été déterminé comme la valeur normale standard.

### B.3. Groupes de patients

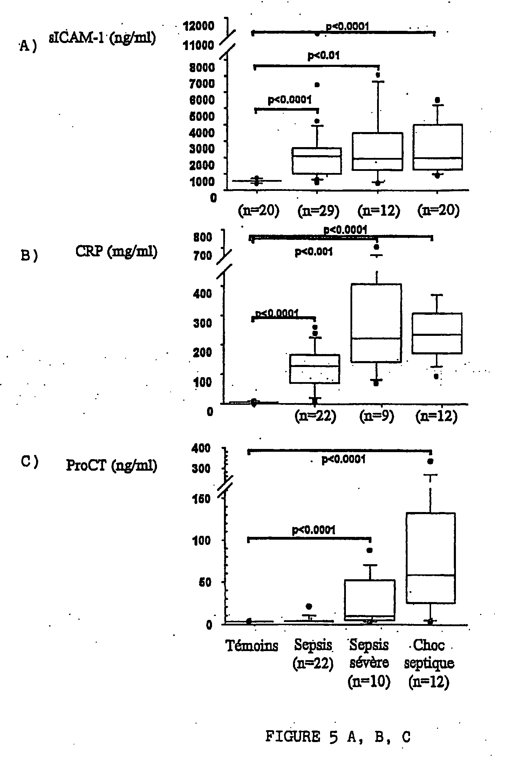

Les résultats des marqueurs circulant dans les groupes de patients sont résumés dans le tableau 1 et illustrés dans les figures 4 et 5.

Dans le sérum des patients admis pour un sepsis sévère, les niveaux de sESM-1, sICAM-1, CRP et proCT sont fortement augmentés pour chaque groupe de patients, comparés au groupe témoin (tableau 1).

Ces niveaux accrus sont statistiquement significatifs (p < à 0,01 ou moins) pour tous les marqueurs et pour tous les groupes de patients, à l'exception de la procalcitonine dans le groupe de patients atteints de sepsis (tableau 1, figures 4 et 5).

La gravité de la maladie est uniquement définie selon trois niveaux graduels désignés respectivement sepsis, sepsis sévère et choc septique. Dans chacun de ces groupes, les niveaux de ESM-1 augmentent de manière statistiquement corrélée avec la gravité de la maladie (p < à 0,01, n = 61) (voir tableau 2).

Des augmentations similaires ont été retrouvées pour les niveaux de procalcitonine sérique et de CRP (respectivement p = 0,0001, n = 34 et p < à 0,01, n = 43).

D'autre part, le niveau de sICAM-1 est augmenté d'environ 5 fois la valeur normale, mais présente un niveau similaire dans chacun des groupes de patients.

Les niveaux de CRP (Anova et Mann-Whitney) et de ProCT. (Mann-Whitney) permettent de distinguer les groupes de patients atteints de sepsis des patients atteints de sepsis sévère, ce qui n'est pas le cas pour les niveaux de ESM-1.

Les niveaux de ESM-1 (Anova et Mann-Whitney) et de ProCT (Mann-Whitney) permettent de distinguer les groupes de patients atteints respectivement de sepsis sévère et de choc septique, ce qui n'est pas le cas pour les niveaux de CRP (voir tableau 2).

Au vu de ces résultats, le niveau de CRP apparaît comme le marqueur le plus sensible dans la maladie la moins sévère et le niveau de ESM-1 apparaît être un meilleur marqueur que le marqueur ProCT pour les sepsis plus sévères.

Une recherche de corrélation entre les niveaux sériques de ESM-1, sICAM-1, CRP ou ProCT a permis d'établir une corrélation significative entre le niveau de CRP et de procalcitonine (p < à 0,01, tableau 3).

Cependant, il n'a été trouvé aucune corrélation entre les autres marqueurs (voir tableau 3).

De plus, aucune corrélation n'a été observée entre les niveaux de ESM-1 et de créatinine plasmatique dans le groupe des patients affectés de chocs septiques (résultats non présentés).

Afin de déterminer si les niveaux sériques de ESM-1 pouvaient prédire un pronostic vital, on a comparé, dans une première approche, les niveaux moyens de ESM-1 sérique entre les patients ayant décédé et les patients ayant survécu.

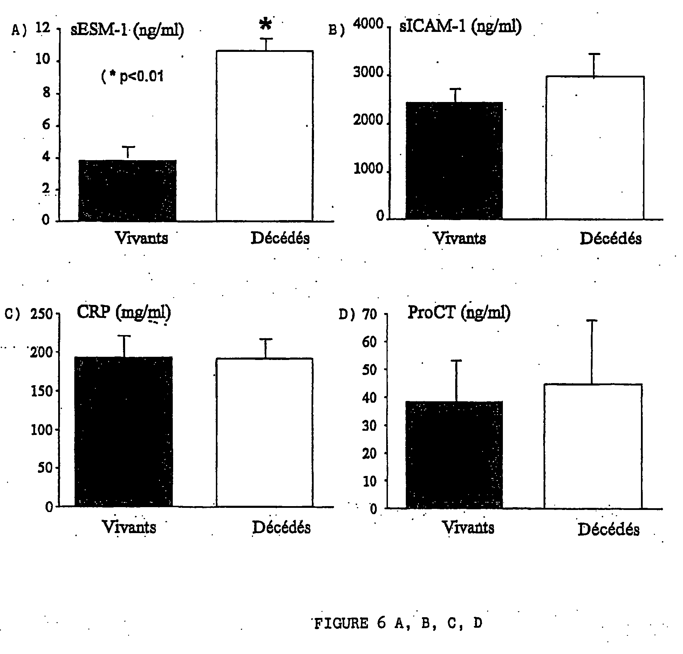

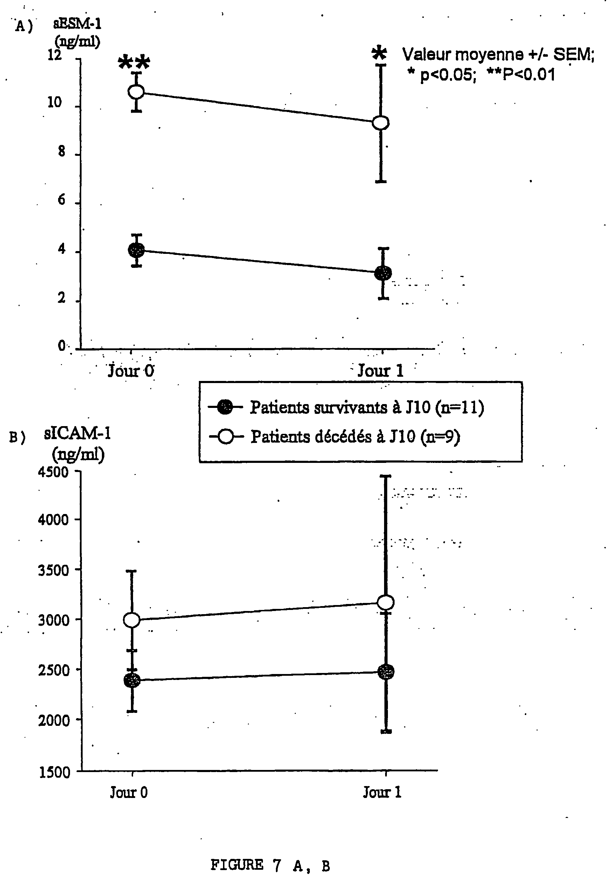

Lorsque tous les patients sont pris en compte dès l'admission en réanimation et selon l'évolution fatale ou non, les résultats montrent que le niveau de ESM-1 sérique au jour d'hospitalisation (jour 0) est significativement plus grand dans le groupe des patients ayant décédé au jour 10 (jour 10; p < à 0,01; figure 6A et figure 7A). On peut souligner que de telles différences ne sont pas mises en évidence avec des autres marqueurs sICAM-1, CRP ou ProCT (figures 6B-9).

Selon une seconde approche, les niveaux de sESM-1 et sICAM1 ont été mesurés à la fois le jour de l'hospitalisation (jour 0) et 24 heures après (jour 1). Le niveau de ESM-1 au jour 0 est significativement plus grand chez des patients ayant décédé que chez les patients ayant survécu (figures 7A). Cette différence persiste au jour 1 (n = 20, p < à 0,05) (figure 7A).

Une telle différence ne peut pas être observée avec aucun des autres marqueurs sICAM-1 (figure 7B) ni avec la CRP, la procalcitonine ou la sICAM-1.

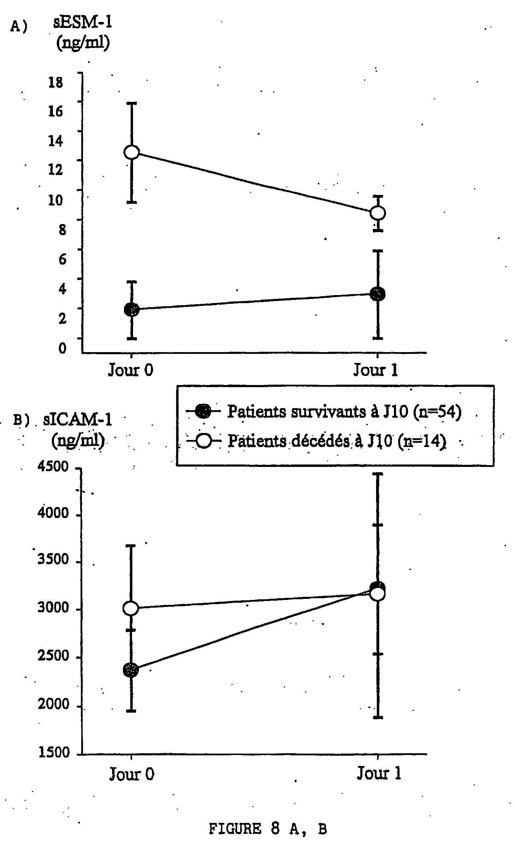

Dans le groupe unique des patients atteints de choc septique, les niveaux sériques de ESM-1 au jour 0 et au jour 1 (n = 11) sont significativement plus grands chez les patients ayant décédé que chez les patients ayant survécu (figures 8A-B).

Dans chaque groupe séparément, aucune corrélation statistiquement significative n'a été trouvée entre les niveaux de sICAM-1 au jour 0, de sICAM-1 au jour 1 (figure 8B), de CRP au jour O, ou de ProCT au jour 0 et la survie au jour 10 de chaque patient.

Les résultats présentés dans cet exemple montrent que la valeur moyenne de la concentration de la protéine ESM-1 circulante est très augmentée par rapport aux sujets sains. Cette augmentation de la concentration moyenne de ESM-1 circulante est corrélée avec le niveau de sévérité clinique de la maladie. Les plus hauts niveaux de ESM-1 circulante au stade précoce de la maladie sont associés avec une probabilité plus faible de survie du patient après 10 jours.

En accord avec des études antérieures, les marqueurs circulant tels que la protéine C réactive ou CRP (Morley et al)., la procalcitonine ou ProCT , (ASSICOT M et al.); (MULLER B et al), (WANNER GA et al.) et la protéine soluble ICAM-1 ou sICAM-1 (KAYAL S et al.), (SESSLER C N et al. (1995) SESSLER C N et al.(1993) sont significativement augmentés chez les patients de l'étude également. Les marqueurs CRP et ProCT sont corrélés avec la sévérité clinique des patients.

En revanche, la détermination des niveaux des trois marqueurs CRP, ProCT et sICAM-1 ne permet pas de prédire de pronostic vital, au contraire de la détermination du niveau de la protéine ESM-1 circulante. De plus, la valeur prédictive du niveau sérique de ESM-1 est majoritairement liée à la valeur initiale de ESM-1 sérique au jour 0 d'hospitalisation.

Dans cette étude, l'augmentation du niveau de ESM-1 sérique n'est pas corrélée avec un dysfonctionnement rénale, comme cela a été mesuré par l'augmentation du niveau de créatinine plasmatique. Ce résultat suggère que les niveaux accrus de la protéine ESM-1 dans le sérum chez les patients atteints de chocs septiques provient de tissus pulmonaires altérés ou des cellules des parois vasculaires en état de stress.

En conclusion, les résultats de l'étude présentée dans cet exemple indiquent que ESM-1 représente un nouveau marqueur du dysfonctionnement des cellules endothéliales chez les patients atteints de sepsis.

Associés aux valeurs d'autres paramètres de marqueurs circulants comme les molécules d'adhésion solubles, les cjrtokines, la procalcitonine ou d'autres marqueurs de dysfonctionnement spécifique de certaines cellules, le niveau de la protéine ESM-1 est de nature à fournir une information d'intérêt sur la sévérité clinique et l'issue chez les patients atteints de sepsis.

**TABLEAU 1**

| **Valeurs de concentration de la protéine ESM-1 circulante chez des volontaires sains et dans différents groupes de patients atteints de sepsis** | | | | | |
|---|---|---|---|---|---|
| | | Marqueurs sanguins | | | |
| Groupe | n | sESM-1 | sICAM-14 | CRP | Procalcitonine |
| | | (ng/ml) | (ng/ml) | (mg/l) | (ng/ml) |
| Valeur normale (sain non atopique) | 20 | 0,7±0,1. | 571±168 | < 10 | < 0,5 |
| Ensemble des patients | 61 | 5,4±0,8*** | 2510±261*** | 194±21** | 39,8±12,4*** |
| Sepsis (pulmonaire) | 29 | 2,5±0,3*** | 2336±406*** | 126±15*** | 2,0±1,4 |
| Sepsis sévère | 12 | 4,5±1,4*** | 2696±721* | 292±73** | 23,8±9,2*** |
| Choc septique | 20 | 10,0±1,8*** | 2661±373*** | 245±29*** | 90,8±29,5*** |

| | | | | | |
|---|---|---|---|---|---|
| Les valeurs des marqueurs sanguins sont exprimées comme la valeur moyenne ± SEM (erreur standard). Différence significative entre chaque groupe de patients et la valeur normale (groupe de sujets sains non atopiques): * p < 0,01; ** p < 0,0001; *** p<0,0001. | | | | | |

**TABLEAU 2**

| **Distinction potentielle de la gravité de l'état du patient en fonction du niveau des marqueurs circulants** | | | | |
|---|---|---|---|---|
| Marqueurs présentant une différence significative (ANOVA) | sESM-1 | sICAM-1 | CRP | ProCT |
| ECS et S/S | p<0,01 | NS | NS | NS |
| ECS et S | p<0,0001 | NS | p<0,05 | p<0,01 |
| S/S et S | NS | NS | p<0,01 | NS |
| | | | | |
| Kruskal-Wallis | p<0,01 | NS | p<0,01 | p=0,0001 |
| | | | | |

| Marqueurs présentant une différence significative (Mann-Whitney) | sESM-1 | SICAM-1 | CRP | ProCT |
|---|---|---|---|---|
| ECS et- S/S | p<0,05 | NS | NS | p<0,05 |
| ECS et S | p<0,001 | NS | p<0,01 | p=0,0001 |
| S/S et S | NS | NS | p<0,05 | p<0,01 |

| | | | | |
|---|---|---|---|---|
| NS = non significatif. | | | | |

**TABLEAU 3**

| **Corrélation entre les différentes valeurs des marqueurs sanguins** | | | | | | | |
|---|---|---|---|---|---|---|---|
| Marqueur | sESM-1 | pESM-1 | Age | 1ICAM-1 | CRP | ProCT | Plasma créatinine |
| sESM-1 | - | p<0,0001 | NS | NS | NS | NS | NS |
| sICAM-1 | NS | - | - | - | NS | NS | - |
| CRP | NS | - | - | NS | - | p<0,01 | - |
| ProCT | NS | - | - | NS | p<0,01 | - | - |

| | | | | | | | |
|---|---|---|---|---|---|---|---|
| NS = non significatif. | | | | | | | |

### REFERENCES.

- AMERICAN COLLEGE OF CHEST PHYSICIANS/SOCIETY OF CRITICAL CARE MEDICINE CONSENSUS CONFERENCE (1992).
- ASSICOT M et al., 1993, Lancet, vol.341: 515-518
- BECHARD et al. (2000) J. Vasc. Res., vol.37 (5): 417-425.
-
- ICHINOSE N et al. (1991, In: Fluorometric analysis in Biomedical Chemistry, vol.10, page 110, Chemical Analysis, Winefordner JD et al. editor, John Whiley and Sons, New York)
- KAYAL S et al., 1998, Am. J.Respir. Crit Care Med, vol.157:774-776;
- KOHLER et MIELSTEIN (1975, Nature, vol.256:495)
- KOZBOR et al. (1983, hybridoma, vol.2 (1): 7-16).
- LASSALLE P et al.(1996) The Journal of Biological Chemistry, vol.271 (34): 20.458-20.464).
- LEGER et al., (1997, Hum. Antibodies, Vol.8 (1): 3-16).
- MARTINEAU et al. (1998, J. Mol. Biol., vol.280 (1): 117-127).
- MORLEY et al., Ann. N.Y. Acad. Sci, 1982, vol. 389:406-418),
- MULLER B et al (2000), Crit. Care Med., vol.28:977-983;
- RIDDER et al; (1995, Biotechnology N Y), vol. 13 (3): 255-260).
- REINMANN et al. (1997, AIDS Res. Hum. Retroviruses, vol.13 (11):933-943)
- SESSLER C.N. et al., Am. J. Respir. Crit Care Med., 1995, vol.151:1420-1427;
- SESSLER C.N., 1993, Chest, vol.104 (2): 11S)
- WANNER et al., 2000, Crit. Care Med. , vol 28 (4): 950-957

## Revendications

1. Nécessaire ou trousse de détection de la protéine ESM-1 dans un échantillon, comprenant:
a) un premier anticorps se liant spécifiquement à la région N-terminale de la protéine ESM-1 comprise entre l'acide aminé en position 20 et l'acide aminé en position 78 de la séquence en acides aminés de cette protéine; et
b) un second anticorps se liant spécifiquement à la région C-terminale comprise entre l'acide aminé en position 79 et l'acide aminé en position 184 de la séquence en acides aminés de la protéine ESM-1.

2. Nécessaire de détection selon la revendication 1, **caractérisé en ce que** l'anticorps se liant spécifiquement à la région N-terminale de la protéine ESM-1 a été obtenu après immunisation d'un mammifère avec la protéine ESM-1 recombinante produite par une cellule eucaryote.

3. Nécessaire de détection selon l'une des revendications 1 et 2, **caractérisé en ce que** l'anticorps se liant spécifiquement à la région N-terminale de la protéine ESM-1 est l'anticorps monoclonal produit par la lignée d'hybridome désignée MEC15 déposée à la Collection de Cultures de Micro-organismes de l'Institut Pasteur le 17 Octobre 2000 sous le n° d'accès I-2572.

4. Nécessaire de détection selon l'une des revendications 1 à 3, **caractérisé en ce que** l'anticorps se liant spécifiquement à la région C-terminale de ESM-1 est choisi parmi les anticorps capables de reconnaître l'un des trois déterminants antigéniques suivants:
a) le déterminant AgD1 allant du résidu proline en position 79 jusqu'au résidu cystéine en position 99 de ESM-1;
b) le déterminant antigénique AgD2 allant du résidu sérine en position 119 jusqu'au résidu valine en position 189 de la protéine ESM-1;
c) le déterminant antigénique AgD3 allant du résidu glycine en position 159 jusqu'au résidu arginine en position 184 de la protéine ESM-1.

5. Nécessaire de détection selon la revendication 4, **caractérisé en ce que** l'anticorps se liant spécifiquement à la région C-terminale de la protéine ESM-1 est choisi parmi les anticorps suivants:
a) l'anticorps MEP 21 produit par la lignée d'hybridome déposée auprès de la CNCM le 19 Novembre 1997 sous le n° d'accès I-1944;
b) l'anticorps monoclonal MEP08 produit par la lignée d'hybridome déposée le 19 Novembre 1997 auprès- de la CNCM sous le n° d'accès -1941;
c) l'anticorps monoclonal MEP14 produit par la lignée d'hybridome déposée le 19 NOVEMBRE 1997 auprès de la CNCM sous le n° d'accès I-1942;
d) l'anticorps monoclonal MEP19 produit par la lignée d'hybridome déposée le 19 Novembre 1997 auprès de la CNCM sous le n° d'accès -1943.

6. Nécessaire de détection selon l'une des revendications 1 à 5, **caractérisé en ce que** l'un au moins des deux anticorps est lié de manière covalente à une molécule permettant sa détection directe, ou indirecte.

7. Nécessaire de détection selon l'une des revendications 1 à 6, **caractérisé en ce que** le premier ou le second anticorps est immobilisé sur un support.

8. Nécessaire de détection selon la revendication 7, **caractérisé en ce que** l'anticorps immobilisé sur un support est l'anticorps reconnaissant spécifiquement la région C-terminale de la protéine ESM-1.

9. Procédé de détection de la protéine ESM-1 dans un échantillon, **caractérisé en ce qu'**il comprend les étapes suivantes:
a) mettre en contact l'échantillon à tester avec un premier anticorps se liant spécifiquement sur la région N-terminate de la protéine ESM-1 allant de l'acide aminé en position 20 à l'acide aminé en position 78 de la séquence de cette protéine ou avec un premier anticorps se liant sur la région C-terminale de la protéine ESM-1;
b) mettre en contact le complexe éventuellement formé entre la protéine ESM-1 présente dans l'échantillon et le premier anticorps avec un second anticorps se liant spécifiquement à la région de la protéine ESM-1, choisie parmi la région N-terminale allant de l'acide aminé en position 20 à l'acide aminé en position 78 de la séquence de cette protéine et la région C-terminale, non reconnue par le premier anticorps; et
c) détecter le complexe formé entre la protéine ËSM-1 et le second anticorps.

10. Procédé de détection selon la revendication 9, **caractérisé en ce que** le premier anticorps est immobilisé sur un support.

11. Procédé de détection selon l'une des revendications 9 et 10, **caractérisé en ce que** le premier anticorps est un anticorps se liant spécifiquement sur la région C-terminale de la protéine ESM-1 et le second anticorps est un anticorps se liant spécifiquement sur la région N-terminale de la protéine ESM-1 allant de l'acide aminé en position 20 à l'acide aminé en position 78 de la séquence de cette protéine.

12. Procédé de détection selon l'une quelconque des revendications 9 à 11, **caractérisé en ce que** l'anticorps se liant spécifiquement sur la région N-terminale de la protéine ESM-1 est l'anticorps MEC15 produit par l'hybridome déposé le 17 Octobre 2000 auprès de la CNCM sous le n° d'accès I-2572.

13. Procédé de détection selon l'une quelconque des revendications 9 à 12, **caractérisé en ce que** l'étape c) du procédé est réalisée à l'aide d'un anticorps biotinylé capable de se fixer au second anticorps.

14. Lignée d'hybridome MEC15 déposée à la CNCM le 19 Novembre 1997 sous le n° I-2572.

15. Anticorps monoclonal produit par la lignée d'hybridome MEC15 déposé le 17 Octobre 2000 auprès de la CNCM sous le n° d'accès I-2572.

16. Utilisation d'une trousse de détection selon l'une quelconque des revendications 1 à 8 pour détecter *in vitro* des altérations de la paroi vasculaire endothéliale chez l'homme.

17. Utilisation d'une trousse d'immunodétection selon l'une des revendications 1 à 8 pour la quantification de la protéine ESM-1 *in vitro* chez un patient traité par un composé immunosuppresseur.

18. Utilisation d'une trousse de détection selon l'une quelconque des revendications 1 à 8 pour la quantification *in vitro* de la protéine ESM-1 chez un patient affecté d'un cancer.

## Claims

1. Kit for detection of protein ESM-1 in a sample, comprising :
a) a first antibody specifically binding to the N-terminal region of protein ESM-1 between the amino acid in position 20 and the amino acid in position 78 of the amino acid sequence of this protein; and
b) a second antibody specifically binding to the C-terminal region between the amino acid in position 79 and the amino acid in position 184 of the amino acid sequence of protein ESM-1.

2. Kit for detection according to claim 1, **characterized in that** the antibody specifically binding to the N-terminal region of protein ESM-1 has been obtained by immunization of a mammal with recombinant protein ESM-1 produced by a eukaryotic cell.

3. Kit for detection according to one of claims 1 and 2, **characterized in that** the antibody specifically binding to the N-terminal region of protein ESM-1 is the monoclonal antibody produced by the hybridoma lineage designated MEC 15 filed at the Collection de Cultures de Micro-organismes (CNCM) of the Institut Pasteur on 17 October 2000 under the access number 1-2572.

4. Kit for detection according to one of claims 1 to 3, **characterized in that** the antibody specifically binding to the C-terminal region of ESM-1 is selected from the antibodies liable to recognize one of the three following antigenic determinants:
a) the determinant AgD1 from the proline residue in position 79 up to the cysteine residue in position 99 of ESM-1,
b) the antigenic determinant AgD2 from the serine residue in position 119 up to the valine residue in position 139 of protein ESM-1;
c) the antigenic determinant AgD3 from the glycine residue in position 159 up to the arginine residue in position 184 of protein ESM-1.

5. Kit for detection according to claim 4, **characterized in that** the antibody specifically binding to the C-terminal region of protein ESM-1 is selected from the following antibodies:
a) the antibody MEP 21 produced by the hybridoma lineage filed at the CNCM on 19 November 1997 under the access number I-1944;
b) the monoclonal antibody MEP08 produced by the hybridoma lineage filed on 19 November 1997 at the CNCM under the access number - 1941;
c) the monoclonal antibody MEP14 produced by the hybridoma lineage filed on 19 November 1997 at the CNCM under the access number I-1942;
d) the monoclonal antibody MEP19 produced by the hybridoma lineage filed on 19 November 1997 at the CNCM under the access number - 1943.

6. Kit for detection according to one of claims 1 to 5, **characterized in that** at least one of the two antibodies is covalently linked to a molecule enabling its direct or indirect detection.

7. Kit for detection according to one of claims 1 to 6, **characterized in that** the first or the second antibody is immobilized on a support.

8. Kit for detection according to claim 7, **characterized in that** the antibody immobilized on a support is the antibody specifically recognizing the C-terminal region of protein ESM-1.

9. Method of detection of protein ESM-1 in a sample, **characterized in that** it comprises the following steps :
a) placing the sample to be tested in contact with a first antibody specifically binding to the N-terminal region of protein ESM-1 between the amino acid in position 20 and the amino acid in position 78 of the amino acid sequence of this protein or with a first antibody binding to the C-terminal region of protein ESM-1;
b) placing the complex potentially formed between the protein ESM-1 present in the sample and the first antibody in contact with a second antibody specifically binding to the region of protein ESM-1, selected from the N-terminal region between the amino acid in position 20 and the amino acid in position 78 of the amino acid sequence of this protein or the C-terminal region, not recognized by the first antibody ; and
c) detecting the complex formed between protein ESM-1 and the second antibody.

10. Method of detection according to claim 9, **characterized in that** the first antibody is immobilized on a support.

11. Method of detection according to one of claims 9 and 10, **characterized in that** the first antibody is an antibody specifically binding to the C-terminal region of protein ESM-1 and the second antibody is an antibody specifically binding to the N-terminal region of protein ESM-1 between the amino acid in position 20 and the amino acid in position 78 of the amino acid sequence of this protein.

12. Method of detection according to any one of claims 9 to 11, **characterized in that** the antibody specifically binding to the N-terminal region of protein ESM-1 is the antibody MEC15 produced by the hybridoma filed on 17 October 2000 at the CNCM under the access number 1-2572.

13. Method of detection according to any one of claims 9 to 12, **characterized in that** step c) is performed using a biotinylated antibody liable to fix to the second antibody.

14. Hybridoma lineage MEC15 filed at the CNCM on 19 November 1997 under the number I-2572.

15. Monoclonal antibody produced by the hybridoma lineage MEC15 filed on 17 October 2000 at the CNCM under the access number I-2572.

16. Use of a detection kit according to any one of claims 1 to 8 to detect *in vitro* deteriorations to the endothelial vascular wall in humans.

17. Use of a immunodetection kit according to any one of claims 1 to 8 for the quantification of protein ESM-1 *in vitro* in a patient treated with an immunosuppressant compound.

18. Use of a detection kit according to any one of claims 1 to 8 for the quantification *in vitro* of protein ESM-1 in a patient suffering from cancer.

## Patentansprüche

1. Kit zum Nachweis des Proteins ESM-1 in einer Probe, umfassend:
a) einen ersten Antikörper, der spezifisch an den N-terminalen Bereich des Proteins ESM-1 zwischen der Aminosäure auf Position 20 und der Aminosäure auf Position 78 der Aminosäuresequenz dieses Proteins bindet; und
b) eine zweiten Antikörper, der spezifisch an den C-terminalen Bereich zwischen der Aminosäure auf Position 79 und der Aminosäure auf Position 184 der Aminosäuresequenz des Proteins ESM-1 bindet.

2. Nachweiskit gemäß Anspruch 1, **dadurch gekennzeichnet, dass** der Antikörper, der spezifisch an den N-terminalen Bereich des Proteins ESM-1 bindet, nach der Immunisierung eines Säugers mit dem rekombinanten Protein ESM-1, das durch eine eukaryontische Zelle produziert wurde, erhalten wird.

3. Nachweiskit gemäß einem der Ansprüche 1 und 2, **dadurch gekennzeichnet, dass** der Antikörper, der spezifisch an den N-terminalen Bereich des Proteins ESM-1 bindet, der monoklonale Antikörper ist, der von der als MEC15 bezeichneten Hybridomlinie produziert wird, die am 17. Oktober 2000 unter der Zugriffsnummer 1-2572 bei der Collection de Cultures de Micro-organismes de l'Institut Pasteur hinterlegt wurde.

4. Nachweiskit gemäß einem der Ansprüche 1 bis 3, **dadurch gekennzeichnet, dass** der Antikörper, der spezifisch an den C-terminalen Bereich von ESM-1 bindet, aus den Antikörpern ausgewählt ist, die eine der folgenden drei antigenen Determinanten erkennen können:
a) die Determinante AgD1, die vom Prolinrest auf Position 79 bis zum Cysteinrest auf Position 99 von ESM-1 reicht;
b) die antigene Determinante AgD2, die vom Serinrest auf Position 119 bis zum Valinrest auf Position 139 des Proteins ESM-1 reicht;
c) die antigene Determinante AgD3, die vom Glycinrest auf Position 159 bis zum Argininrest auf Position 184 des Proteins ESM-1 reicht.

5. Nachweiskit gemäß Anspruch 4, **dadurch gekennzeichnet, dass** der Antikörper, der spezifisch an den C-terminalen Bereich des Proteins ESM-1 bindet, aus den folgenden Antikörpern ausgewählt ist:
a) dem Antikörper MEP21, der von der Hybridomlinie produziert wird, die am 19. November 1997 unter der Zugriffsnummer 1-1944 bei der CNCM hinterlegt wurde;
b) dem monoklonalen Antikörper MEP08, der von der Hybridomlinie produziert wird, die am 19. November 1997 unter der Zugriffsnummer 1-1941 bei der CNCM hinterlegt wurde;
c) dem monoklonalen Antikörper MEP14, der von der Hybridomlinie produziert wird, die am 19. November 1997 unter der Zugriffsnummer 1-1942 bei der CNCM hinterlegt wurde;
d) dem monoklonalen Antikörper MEP19, der von der Hybridomlinie produziert wird, die am 19. November 1997 unter der Zugriffsnummer 1-1943 bei der CNCM hinterlegt wurde.

6. Nachweiskit gemäß einem der Ansprüche 1 bis 5, **dadurch gekennzeichnet, dass** wenigstens einer der beiden Antikörper kovalent an ein Molekül gebunden ist, das seinen direkten oder indirekten Nachweis ermöglicht.

7. Nachweiskit gemäß einem der Ansprüche 1 bis 6, **dadurch gekennzeichnet, dass** der erste oder der zweite Antikörper auf einem Träger immobilisiert ist.

8. Nachweiskit gemäß Anspruch 7, **dadurch gekennzeichnet, dass** der auf einem Träger immobilisierte Antikörper der Antikörper ist, der spezifisch den C-terminalen Bereich des Proteins ESM-1 erkennt.

9. Verfahren zum Nachweis des Proteins ESM-1 in einer Probe, **dadurch gekennzeichnet, dass** es die folgenden Schritte umfasst:
a) In-Kontakt-Bringen der zu testenden Probe mit einem ersten Antikörper, der spezifisch an den N-terminalen Bereich des Proteins ESM-1 zwischen der Aminosäure auf Position 20 und der Aminosäure auf Position 78 der Aminosäuresequenz dieses Proteins bindet, oder mit einem ersten Antikörper, der an den C-terminalen Bereich des Proteins ESM-1 bindet;
b) In-Kontakt-Bringen des Komplexes, der gegebenenfalls zwischen dem in der Probe vorhandenen Protein ESM-1 und dem ersten Antikörper gebildet wurde, mit einem zweiten Antikörper, der spezifisch an den Bereich des Proteins ESM-1 bindet, der aus dem N-terminalen Bereich zwischen der Aminosäure auf Position 20 und der Aminosäure auf Position 78 der Aminosäuresequenz dieses Proteins und dem C-terminalen Bereich ausgewählt ist, je nachdem, welcher Bereich vom ersten Antikörper nicht erkannt wurde; und
c) Nachweisen des zwischen dem Protein ESM-1 und dem zweiten Antikörper gebildeten Komplexes.

10. Nachweisverfahren gemäß Anspruch 9, **dadurch gekennzeichnet, dass** der erste Antikörper auf einem Träger immobilisiert ist.

11. Nachweisverfahren gemäß einem der Ansprüche 9 und 10, **dadurch gekennzeichnet, dass** der erste Antikörper ein Antikörper ist, der spezifisch an den C-terminalen Bereich des Proteins ESM-1 bindet, und der zweite Antikörper ein Antikörper ist, der spezifisch an den N-terminalen Bereich des Proteins ESM-1 zwischen der Aminosäure auf Position 20 und der Aminosäure auf Position 78 der Aminosäuresequenz dieses Proteins bindet.

12. Nachweisverfahren gemäß einem der Ansprüche 9 bis 11, **dadurch gekennzeichnet, dass** der Antikörper, der spezifisch an den N-terminalen Bereich des Proteins ESM-1 bindet, der Antikörper MEC15 ist, der von dem Hybridom produziert wird, das am 17. Oktober 2000 unter der Zugriffsnummer 1-2572 bei der CNCM hinterlegt wurde.

13. Nachweisverfahren gemäß einem der Ansprüche 9 bis 12, **dadurch gekennzeichnet, dass** Schritt c) des Verfahrens mit Hilfe eines biotinylierten Antikörpers durchgeführt wird, der an den zweiten Antikörper binden kann.

14. Hybridomlinie MEC15, die am 19. November 1997 unter der Nr. 1-2572 bei der CNCM hinterlegt wurde.

15. Monoklonaler Antikörper, der von der Hybridomlinie MEC15 produziert wird, die am 17. Oktober 2000 unter der Zugriffsnummer 1-2572 bei der CNCM hinterlegt wurde.

16. Verwendung eines Nachweiskits gemäß einem der Ansprüche 1 bis 8 zum Nachweisen der Veränderungen der endothelialen Gefäßwand beim Menschen in vitro.

17. Verwendung eines Immunnachweiskits gemäß einem der Ansprüche 1 bis 8 zur Quantifizierung des Proteins ESM-1 in vitro bei einem Patienten, der mit einer immunsupprimierenden Verbindung behandelt wurde.

18. Verwendung eines Nachweiskits gemäß einem der Ansprüche 1 bis 8 zur Quantifizierung des Proteins ESM-1 in vitro bei einem Patienten, der unter einer Krebserkrankung leidet.
